# EUROPEAN PATENT SPECIFICATION

(11) **EP 3 004 889 B1**
(45) Date of publication and mention of the grant of the patent: **24.07.2019**
(21) Application number: 14726744.7
(22) Date of filing: 22.05.2014
(51) Int. Cl.: G01N 33/68, G01N 33/92

(54) **METHOD FOR DETERMINING CARDIOVASCULAR DISEASE RISK BY DETERMINING THE LEVEL OF AT LEAST TWO OF THE LIPIDS OF TRIACYLGLYCEROL, CHOLESTEROL ESTERS AND PHOSPHATIDYLETHANOLAMINE**
VERFAHREN ZUM BESTIMMEN DES RISIKOS EINER KARDIOVASKULÄREN ERKRANKUNG DURCH DAS BESTIMMEN DES GEHALTS VON MINDESTENS 2 DER LIPIDE TRIACYLGLYCERIN, CHOLESTERINESTER UND PHOSPHATIDYLETHANOLAMIN
METHODE POUR DETERMINER LE RISQUE D'UNE MALADIE CARDIOVASCULAIRE PAR LA DETERMINATION DE LA QUANTITE D'AU MOINS 2 DES LIPIDES TRIACYLGLYCEROL, ESTER DE CHOLESTEROL ET PHOSPHATIDYLETHAOLAMINE

(30) Priority: 24.05.2013 GB 201309426
(43) Date of publication of application: 13.04.2016
(73) Proprietor: King's College London, London WC2R 2LS (GB)
(72) Inventor: MAYR, Manuel, London WC2R 2LS (GB)
(74) Representative: Tombling, Adrian George
(86) International application number: PCT/GB2014/051578
(87) International publication number: WO 2014/188202

(56) References cited:
- EP-A1- 1 385 003
- WO-A1-2011/063470
- WO-A2-2006/031963
- WO-A2-2012/151039
- WO-A2-2012/166798
- GOTTO A M: "HIGH-DENSITY LIPOPROTEIN CHOLESTEROL AND TRIGLYCERIDES AS THERAPEUTIC TARGETS FOR PREVENTING AND TREATING CORONARY ARTERY DISEASE", AMERICAN HEART JOURNAL, MOSBY- YEAR BOOK INC, US, vol. 144, no. 6, SUPPL, 1 December 2002 (2002-12-01), pages S33-S42, XP008027531, ISSN: 0002-8703, DOI: 10.1067/MHJ.2002.130301
- M. J. CHAPMAN ET AL: "Triglyceride-rich lipoproteins and high-density lipoprotein cholesterol in patients at high risk of cardiovascular disease: evidence and guidance for management", EUROPEAN HEART JOURNAL, vol. 32, no. 11, 29 April 2011 (2011-04-29), pages 1345-1361, XP055133273, ISSN: 0195-668X, DOI: 10.1093/eurheartj/ehr112
- CECILE CHAMBRIER ET AL: "Medium-and Long-Chain Triacylglycerols in Postoperative Patients: Structured Lipids Versus a Physical Mixture", NUTRITION, vol. 15, no. 4, 1 January 1999 (1999-01-01), pages 274-277, XP055116198, ISSN: 0899-9007
- CAZITA P M ET AL: "Reversible flow of cholesteryl ester between high-density lipoproteins and triacylglycerol-rich particles is modulated by the fatty acid composition and concentration of triacylglycerols.", BRAZILIAN JOURNAL OF MEDICAL AND BIOLOGICAL RESEARCH = REVISTA BRASILEIRA DE PESQUISAS MÉDICAS E BIOLÓGICAS / SOCIEDADE BRASILEIRA DE BIOFÍSICA ... [ET AL.] DEC 2010, vol. 43, no. 12, December 2010 (2010-12), pages 1135-1142, XP002728203, ISSN: 1414-431X
- STEGEMANN CHRISTIN ET AL: "Lipidomics profiling and risk of cardiovascular disease in the prospective population-based Bruneck study.", CIRCULATION 6 MAY 2014, vol. 129, no. 18, 6 May 2014 (2014-05-06), pages 1821-1831, XP002728153, ISSN: 1524-4539

## Description

### FIELD OF THE INVENTION

The present invention relates in general to the field of biomarkers indicative of cardiovascular disease (CVD) risk. In particular, the present invention relates to a method of determining CVD risk by measuring the levels of such biomarkers, and to associated methods of treatments and kits.

### BACKGROUND OF THE INVENTION

Cardiovascular disease (CVD) is a major cause of mortality and morbidity throughout the world, and represents one of the most significant challenges to healthcare systems particularly in industrialized countries. The term CVD covers numerous conditions that affect the heart and vasculature of the body, including coronary artery disease and stroke. A common mechanism underlying many types of CVD is atherosclerosis, the thickening of the walls of arterial blood vessels due to the deposition of lipid-containing plaques. Plasma lipids such as cholesterol and triglyceride are thought to play a key role in the development of atherosclerosis.

Since alterations in plasma lipid profiles may be linked to atherosclerosis, the measurement and management of plasma or serum lipid concentrations has been used in the prevention and treatment of patients thought to be at risk of CVD. Typically concentrations of triglycerides, total cholesterol, LDL-cholesterol or HDL-cholesterol in plasma may be used for CVD risk prediction. Current treatment strategies tend to focus on reducing LDL-cholesterol concentrations (mainly by statin treatment) or raising HDL-cholesterol (e.g. using CETP-inhibitors).

However, such measurements do not always correlate well with the risk of a subject experiencing an acute ischemic event such as a heart attack or stroke. Many patients who experience an acute myocardial infarction have cholesterol, triglyceride and/or LDL-cholesterol levels within a normal range. Moreover, there is a substantial residual risk of myocardial infarction in statin-treated patients despite a reduction in LDL-cholesterol levels. Recent studies have suggested that the type of LDL particle (i.e. its size and lipid content) may be a more relevant indicator of CVD risk than overall LDL cholesterol levels. Thus the existing strategies for managing CVD risk may fail to accurately identify all patients at high risk, potentially resulting in lack of preventative therapy and avoidable mortality, while others are wrongly classified as high risk and receive unnecessary treatment. More refined strategies for the prediction and management of CVD are therefore required.

In this context, there is a particular need for new biomarkers which can more accurately predict CVD risk. In particular, there is a need for a method of determining the risk of experiencing an acute ischemic event such as myocardial infarction or stroke. Such a method would enable the early, targeted initiation of prophylactic therapy to prevent the development of potentially fatal conditions, and would be of great benefit in reducing mortality and morbidity in patients suffering from CVD.

One potential reason for the lack of specificity of existing methods is that the plasma lipid measurements which are currently used fail to take into account the significantly varying roles of individual lipids in the development of atherosclerosis. Atherosclerotic plaques are complex molecular formations that contain numerous individual lipid species. Moreover, acute ischemic events such as myocardial infarction and stroke typically result from plaque destabilization and rupture, as well as thrombosis on surface of plaques, rather than the development of plaques per se.

There is therefore a need for biomarkers which are more indicative of the tendency of atherosclerotic plaques to rupture and/or to result in thrombosis, leading in arterial occlusion and potentially fatal events. Such biomarkers would be more accurate predictors of CVD risk, particularly in the long term, e.g. over a period of up to ten years. They would thus be highly useful for stratifying patients according to CVD risk and for improving intervention strategies based on therapeutic treatment, as well as nutritional and lifestyle changes which may benefit the patient over an extended period of time.

WO 2011/063470 discloses a variety of lipid biomarkers for stable and unstable heart disease.

### SUMMARY OF THE INVENTION

Accordingly, in one aspect the present invention provides a method of determining cardiovascular disease (CVD) risk in a subject as defined in appended claims 1 to 10.

In a further aspect, the present invention provides a method for preventing or treating cardiovascular disease in a subject, comprising (a) determining CVD risk in the subject by a method as defined above; and (b) administering an anti-CVD therapeutic, wherein the anti-CVD therapeutic agent is an HMG CoA reductase inhibitor, e.g. a statin.

### BRIEF DESCRIPTION OF THE DRAWINGS

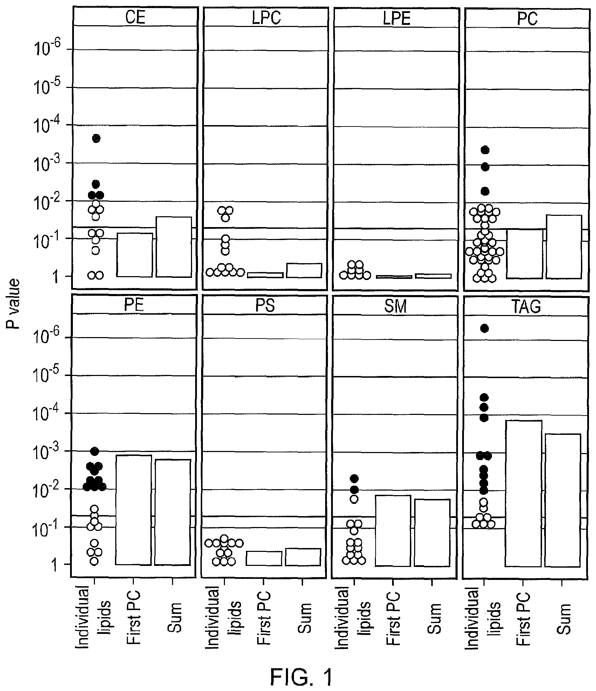

Figure 1: Manhattan plot depicting significance levels (logarithmic y-axis) for the association of individual lipid species and lipid groups with incident CVD. The composite CVD endpoint considers myocardial infarction, ischemic stroke, and sudden cardiac death (Bruneck Study 2000-2010, 90 events). Individual lipids are visualized as dots and lipid groups as bars ("First PC" corresponds to the first principal component; "Sum" denotes the sum of all lipids within a group). Lipid classes are shown in separate panels. P values were derived from Cox proportional hazards models including the respective lipid species, age, sex, and statin therapy. Lipids maintaining significance after adjustment for multiple testing by the Benjamini-Hochberg procedure, controlling the false-discovery rate at the 0.05 level, are marked with black dots. CE, cholesteryl ester; LPC, lysophosphatidylcholine; LPE, lysophosphatidylethanolamine; PC, phosphatidylcholine; PE, phosphatidylethanolamine; PS, phosphatidylserine; SM, sphingomyelin; TAG, triacylglycerol.

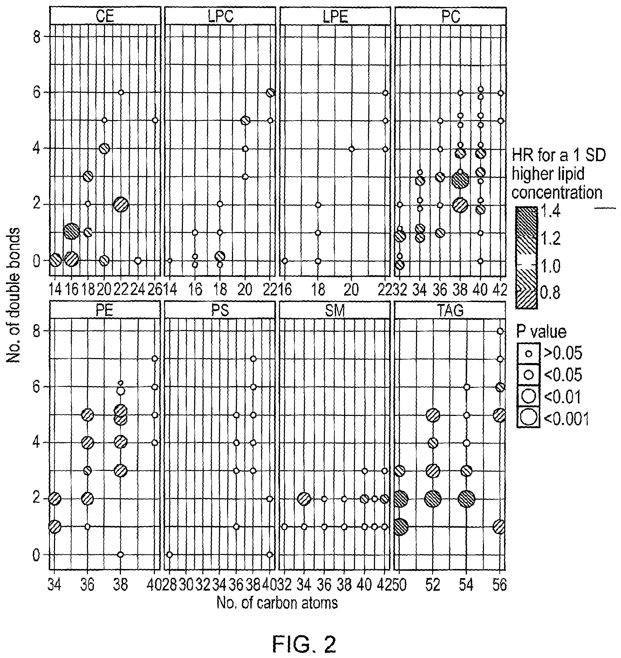

**Figure 2****: Associations of 135 lipid species with incident CVD.** The composite CVD endpoint considers myocardial infarction, ischemic stroke and sudden cardiac death (Bruneck Study 2000-2010, n=90 events). Individual lipid species are depicted by filled circles and arranged by lipid class in eight panels according to the number of total carbon atoms (x-axes) and number of double bonds (y-axes). Circle shade indicates the magnitude of hazard ratio (HR) and circle size corresponds to the significance level (see legend). HRs were calculated for a 1-SD unit higher lipid concentration and derived from Cox proportional hazards models, adjusting for age, sex, and statin therapy. Lipids with the same number of carbon atoms and double bonds are pulled apart vertically to increase their visibility. The distinguishing feature in this case is the presence of an alkyl ether linkage, signified in the formula as e.g. PC(**O-**38:3). Lipids possessing such a linkage are pulled upwards, their alkyl-ether free counterparts downwards. CE, cholesteryl ester; LPC, lysophosphatidylcholine; LPE, lysophosphatidylethanolamine; PC, phosphatidylcholine; PE, phosphatidylethanolamine; PS, phosphatidylserine; SM, sphingomyelin; TAG, triacylglycerol.

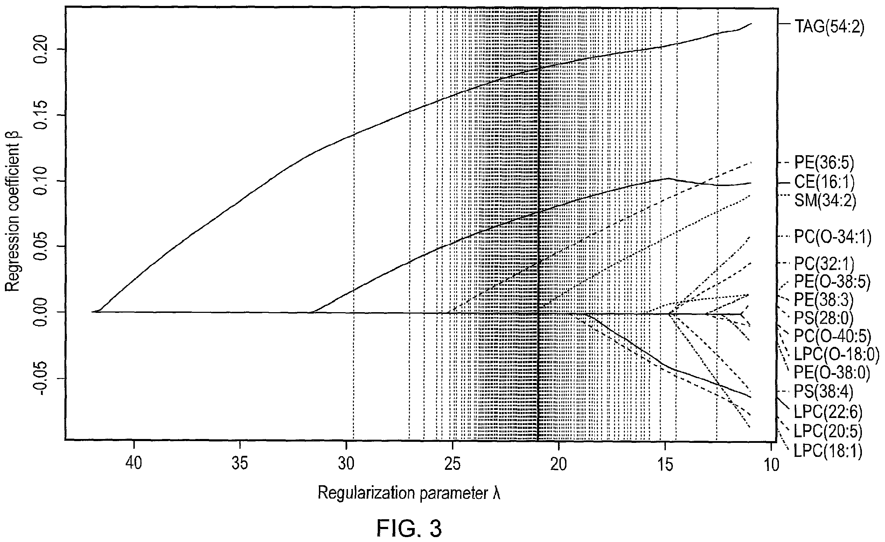

**Figure 3****: Identification of the best-fitting lipid species according to L1- regularized Cox regression analysis.** The graph shows the LASSO (Least absolute shrinkage and selection operator method) regularization path and visualizes regression coefficients of the individual lipid species (y-axis) with varying degree of regularization (x-axis). Coefficients were derived from an L1-regularized Cox proportional hazards model with age, sex, and statin medication treated as unpenalized and the 135 individual lipid species as penalized explanatory variables. The optimal lambda was determined by ten-fold partial likelihood cross-validation. Estimation of optimal lambda was repeated one thousand times and the percentiles (0th, 1st, 2nd, 3rd, 4th, ... , 99th, 100th) of the resulting distribution of lambdas are shown as dashed grey vertical lines. The mean optimal lambda is shown as a solid black vertical line. Variables selected are summarized in **Table 1.**

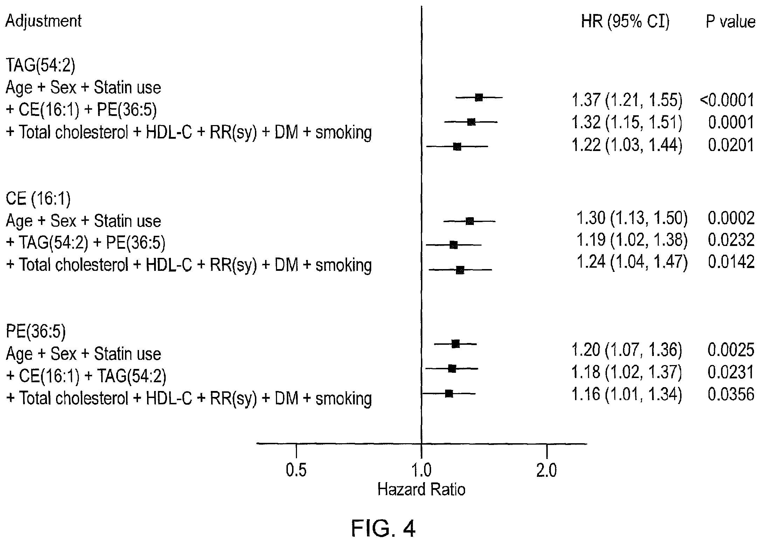

**Figure 4****: Multivariable effect estimates and confidence intervals for the three lipid species most consistently associated with incident CVD.** The composite CVD endpoint considers myocardial infarction, stroke and sudden cardiac death (Bruneck Study, n=90). HRs (95%Cl) were derived from standard Cox regression models with progressive adjustment and calculated for a 1-SD higher lipid levels. HR, hazard ratio; Cl, confidence interval; TAG, triacylglycerol; CE, cholesteryl ester; PE, phosphatidylethanolamine; HDL-C, high-density lipoprotein cholesterol; RR(sy), systolic blood pressure; DM, diabetes mellitus.

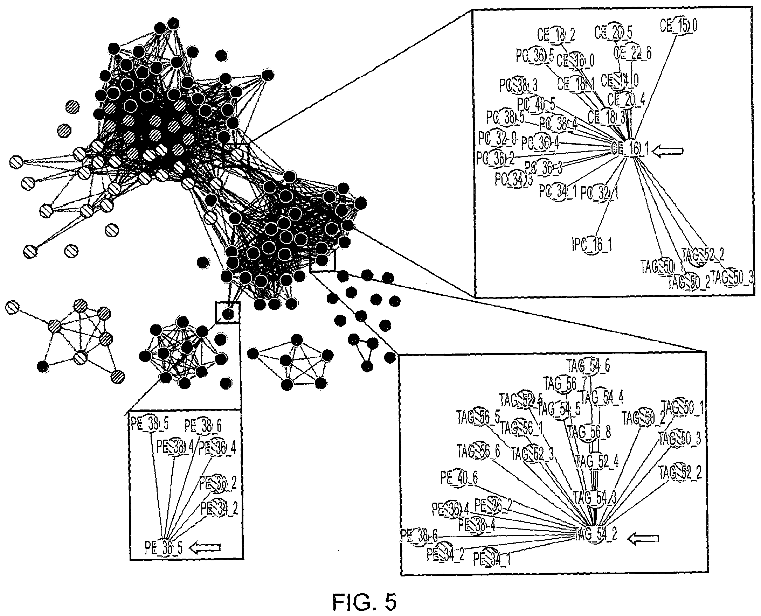

**Figure 5****: Lipid network analysis.** The lipid correlation network, thresholded at an adjacency of 0.02 (akin weighted correlation of 0.8), is shown. The shades indicate the modules detected by Topological Overlap Measure (TOM). While many of the lipid species within the same class belong to the same module, there are modules, which contain more than one lipid class. The direct neighbors of three lipid species - CE(16:1), TAG(54:2) and PE(36:5) - are shown in the boxed area. Lipid species associated with CVD in the Bruneck study (after multiple testing corrections) are depicted as shaded nodes. Lipid molecules directly connected to either CE(16:1) and TAG(54:2) or PE(36:5) and TAG(54:2) are highlighted by shaded edges.

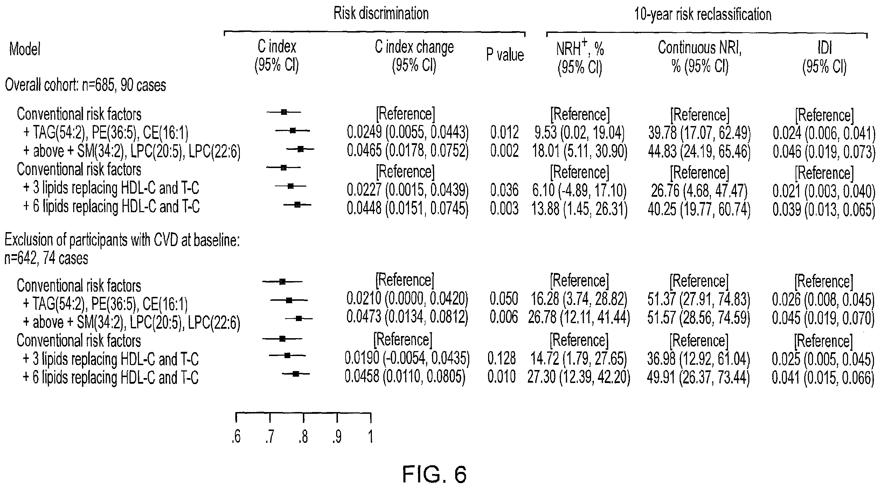

**Figure 6****: Lipidomics to improve CVD risk discrimination and classification into 10-year risk categories.** Reclassification metrics of 10-year CVD risk were calculated across risk categories <5%, 5-10%, 10-20% and >20%. Conventional risk factors are the classic Framingham Risk Score components age, sex, diabetes, smoking status, systolic blood pressure, total cholesterol and HDL cholesterol. The number of cases and non-cases that contributed to the calculation of 10-year risk NRIs was: 90 and 498 for the analysis "overall cohort"; and 74 and 483 for the analysis that excluded participants with CVD at baseline. HDL-C, high-density lipoprotein cholesterol; T-C, total cholesterol.

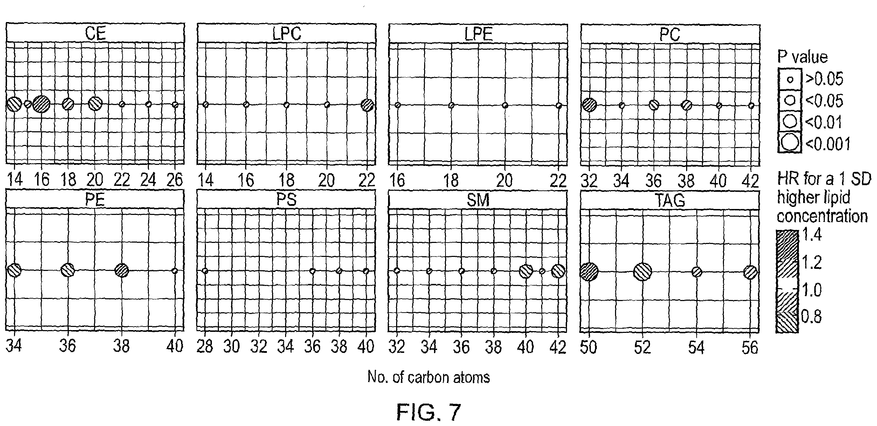

**Figure 7****: Association of fatty acid chain length to CVD risk for different lipid classes.** Circle shade indicates the magnitude of hazard ratio (HR) and circle size corresponds to the significance level (see legend). HRs are aggregated by carbon number for each lipid class, i.e. species comprising alkyl ether linkages are not separately analysed for each carbon number.

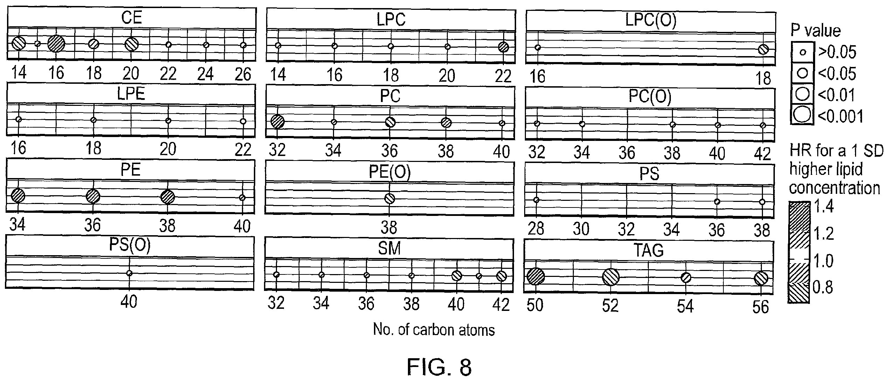

**Figure 8****: Association of fatty acid chain length to CVD risk for different lipid classes, distinguished based on presence of alkyl ether linkages.** Circle shade indicates the magnitude of hazard ratio (HR) and circle size corresponds to the significance level (see legend). HRs are aggregated by carbon number for each lipid class, and then further classified according to whether an alkyl ether linkage (O) is present.

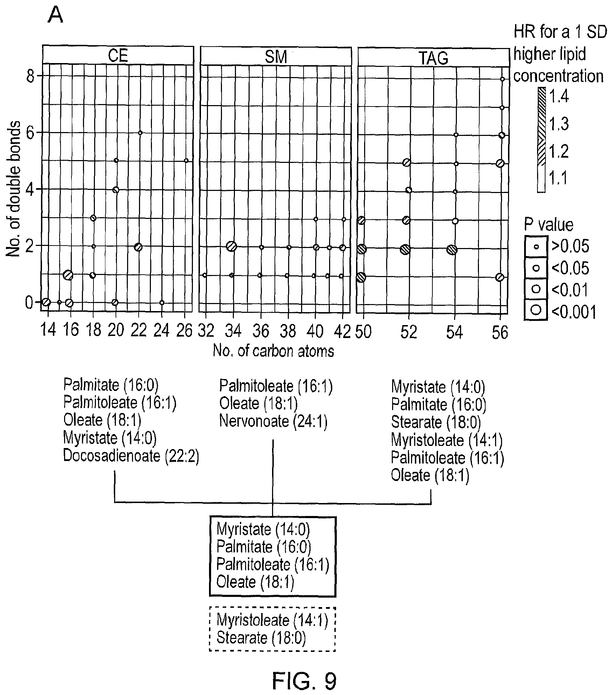

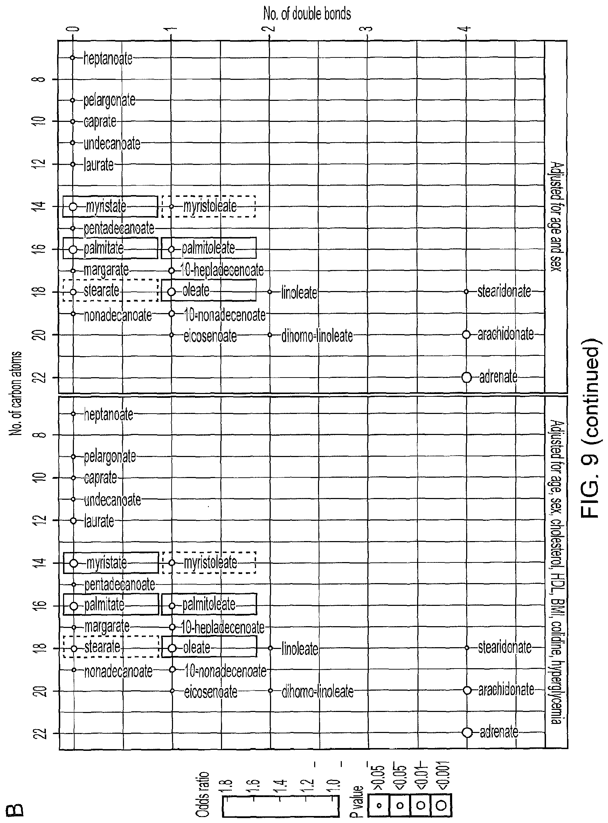

**Figure 9****: Replication in the TwinsUK Cohort.** A) FA composition of lipid species associated with CVD in the Bruneck Study from the CE, SM and TAG classes, which accounted for more than 80% of plasma lipids and thus dominated the FA pool in circulation. This figure depicts the most abundant FA in the respective groups considering the quantity of the individual lipid species. FA composition in TAGs was estimated by a simulation analysis considering all combinations of long chain FA that resulted in the correct carbon and double bond number. B) Associations of plasma FA with CVD in the TwinsUK Cohort (n=1453, 45 cases) with adjustment for age and sex only (left panel) or multivariate adjustment for age, sex, total cholesterol, HDL cholesterol, body-mass index, current smoking (ascertained by cotidine levels), and diabetes (right panel). The key selection of FA in the Bruneck Study is highlighted. Bold numbers are the lipid species with the highest concentration.

### DETAILED DESCRIPTION OF THE INVENTION

### Determining cardiovascular disease risk

The present invention relates in one aspect to a method of determining cardiovascular disease (CVD) risk in a subject. For instance, the method may be used to predict the likelihood of CVD developing in the subject in the future, or to assess the current extent of CVD progression in the subject. The method may also be used to assess the efficacy of an anti-CVD intervention, for instance to monitor the effectiveness of a therapeutic treatment for CVD. Preferably the method is used to predict the long-term risk of CVD, for instance in a period of up to 10 years from when the sample was obtained.

The CVD is preferably associated with atherosclerosis. The CVD may be, for example, coronary artery disease, angina pectoris, congestive heart failure, hypertension, cerebrovascular disease, stroke, myocardial infarction, deep vein thrombosis, peripheral artery disease, cardiomyopathy, cardiac arrhythmia, aortic stenosis or aneurysm.

In a more preferred embodiment, the CVD is associated with thrombosis resulting from or rupture of atherosclerotic plaques. For instance, the method is used to predict the risk of an acute ischemic event occurring in the subject. The acute ischemic event may be, for example, an acute myocardial infarction or an ischemic stroke, or sudden cardiac death. The method may be used to indicate long-term risk of the occurrence of such ischemic events. For instance, in one embodiment the method provides an indication of the risk of an acute ischemic event occurring within a period of up to 10 years (e.g. 6 months to 10 years, 1 year to 10 years, or 5 years to 10 years after the date of the sample).

### Subject

The present method may be carried out on any subject, including non-human or human subjects. In one embodiment, the subject is a mammal, preferably a human. The subject may alternatively be a non-human mammal, including for example a dog, cat, horse, cow, sheep or pig.

In one embodiment, the subject may be suspected of suffering from CVD, or may already be known to have developed CVD. For instance, the subject may be known to be suffering from a condition which may be associated with elevated CVD risk, such as diabetes mellitus. In such embodiments, the method may be performed to assess the extent or severity of CVD and/or to monitor the effectiveness of therapy. Alternatively, the subject may be otherwise healthy and have no known history of or predetermined susceptibility to CVD. For instance, normal individuals may be screened for CVD risk using the method as part of a routine check-up or examination.

The subject may be of any age, including children, young adults and elderly individuals. Preferably the subject is in the age range 30 to 100 years, more preferably 40 to 80 years. The subject may be male or female.

### Sample

The present method comprises a step of determining the level of one or more lipids in a sample obtained from a subject. Thus the present method is typically practiced outside of the human or animal body, e.g. on a body fluid sample that was previously obtained from the subject to be tested. Preferably the sample is derived from blood, i.e. the sample comprises whole blood or a blood fraction. Most preferably the sample comprises blood plasma or serum.

Techniques for collecting blood samples and separating blood fractions are well known in the art. For instance, vena blood samples can be collected from patients using a needle and deposited into plastic tubes. The collection tubes may, for example, contain spray-coated silica and a polymer gel for serum separation. Serum can be separated by centrifugation at 1300 RCF for 10 min at room temperature and stored in small plastic tubes at -80°C.

### Determining lipid levels in the sample

The levels of individual lipid species in the sample may be determined by any suitable method. For example, nuclear magnetic resonance spectroscopy (¹H-NMR) or mass spectroscopy (MS) may be used. Other spectroscopic methods, chromatographic methods, labeling techniques, or quantitative chemical methods may be used in alternative embodiments. Most preferably, the lipid levels in the sample are determined by mass spectroscopy. Typically the lipid level in the sample and the reference value are determined using the same analytical method.

### Lipids

The present method involves determining the levels of one or more lipids selected from triacylglycerols (TAGs), cholesterol esters (CEs) and phosphatidylethanolamines (PEs). In some embodiments, the level of one or more phosphatidylcholines (PCs) may be determined in the sample. The present inventors have surprising found that compared to measurements of overall TAG, CE, PE or PC levels, specific individual lipid species within these groups are much more accurate predictors of CVD risk, especially the long-term risk of experiencing an acute ischemic event.

### Triacylglycerols

In one embodiment, a triacylglycerol (TAG) from TAG(50:1) to TAG(54:5) is determined. In the nomenclature (X:Y), X refers to the total number of carbon atoms in the fatty acid portions of the molecule, and Y defines the total number of double bonds in the fatty acid portions of the molecule. Thus a triacylglycerol (TAG) from TAG(50:1) to TAG(54:5) refers to a TAG comprising 50 to 54 carbon atoms in the fatty acid chains and 1 to 5 double bonds in the fatty acid chains. The present method involves determining the level of one or more such individual species of TAG.

In a preferred embodiment, the TAG comprises 50, 52 or 54 carbon atoms in the fatty acid chains. In this embodiment, the TAG preferably comprises a total of 2 double bonds in the fatty acid portions of the molecule. For instance, a triacylglycerol from TAG(50:2) to TAG(54:2) may be determined.

In another embodiment, the TAG comprises 50 carbon atoms in the fatty acid chains, and preferably 1 to 3 double bonds in the fatty acid portions. For instance, levels of a triacylglycerol from TAG(50:1) to TAG(50:3) may be determined.

In another embodiment, the TAG comprises 52 carbon atoms in the fatty acid chains, and preferably 2 to 5 double bonds in the fatty acid portions. For instance, levels of a triacylglycerol from TAG(52:2) to TAG(52:5) may be determined.

In another embodiment, the TAG comprises 54 carbon atoms in the fatty acid chains, and preferably 2 to 4 double bonds in the fatty acid portions. For instance, levels of a triacylglycerol from TAG(54:2) to TAG(54:4) may be determined.

In accordance with the claimed invention the triacylglycerol to be determined is selected from the group consisting of TAG(50:1), TAG(50:2), TAG(50:3), TAG(52:2), TAG(52:3), TAG(52:4), TAG(52:5), TAG(54:2) and TAG(54:3). More preferably the triacylglycerol is selected from TAG(50:1), TAG(50:2), TAG(52:2) and TAG(54:2). Most preferably the triacylglycerol is TAG(54:2).

### Cholesterol esters

In one embodiment, a cholesterol ester (CE) from CE(14:0) to CE(16:1) is determined (using the nomenclature (X:Y) as defined above). Thus a cholesterol ester (CE) from CE(14:0) to CE(16:1) refers to a CE comprising 14 to 16 carbon atoms in the fatty acid chain and 0 or 1 double bonds in the fatty acid chain. The present method involves determining the level of one or more such individual species of CE.

In a preferred embodiment, the CE comprises 14 carbon atoms in the fatty acid portion of the molecule, and preferably 1 double bond in the fatty acid portion. For instance, levels of CE(14:0) may be determined.

In another embodiment, the CE comprises 16 carbon atoms in the fatty acid chain, and preferably 0 or 1 double bond in the fatty acid portion. For instance, levels of CE(16:0) or CE(16:1) may be determined.

In accordance with the claimed invention the cholesterol ester to be determined is selected from the group consisting of CE(14:0), CE(16:0) and CE(16:1). Most preferably the cholesterol ester is CE(16:1).

### Phosphatidylethanolamines

In one embodiment, a phosphatidylethanolamine (PE) from PE(34:1) to PE(38:6) is determined (using the nomenclature (X:Y) as defined above). Thus a phosphatidylethanolamine (PE) from PE(34:1) to PE(38:6) refers to a PE comprising 34 to 38 carbon atoms in the fatty acid chains and 1 to 6 double bonds in the fatty acid chains. The present method involves determining the level of one or more such individual species of PE.

In one embodiment, the PE comprises 34 carbon atoms in the fatty acid chains, and preferably 1 or 2 double bonds in the fatty acid portions. For instance, levels of PE(34:1) or PE(34:2) may be determined.

In another embodiment, the PE comprises 36 carbon atoms in the fatty acid chains, and preferably 2 to 5 double bonds in the fatty acid portions. For instance, levels of a phosphatidylethanolamine from PE(36:2) to PE(36:5) may be determined.

In another embodiment, the PE comprises 38 carbon atoms in the fatty acid chains, and preferably 3 to 6 double bonds in the fatty acid portions. For instance, levels of a phosphatidylethanolamine from PE(38:3) to PE(38:6) may be determined.

In accordance with the claimed invention the phosphatidylethanolamine to be determined is selected from the group consisting of PE(34:1), PE(34:2), PE(36:2), PE(36:3), PE(36:4), PE(36:5), PE(38:3), PE(38:4), PE(38:5) and PE(38:6). More preferably the phosphatidylethanolamine is PE(36:5).

### Phosphatidylcholine

In one embodiment, a phosphatidylcholine (PC) from PC(32:0) to PC(40:4) is determined (using the nomenclature (X:Y) as defined above). Thus a phosphatidylcholine (PC) from PC(32:0) to PC(40:4) refers to a PC comprising 32 to 40 carbon atoms in the fatty acid chains and 0 to 4 double bonds in the fatty acid chains. The present method involves determining the level of one or more such individual species of PC.

In one embodiment, the PC comprises 32 carbon atoms in the fatty acid chains, and preferably 0 or 1 double bond in the fatty acid portions. For instance, levels of PC(32:0) or PC(32:1) may be determined.

In another embodiment, the PC comprises 34 carbon atoms in the fatty acid chains, and preferably 1 to 3 double bonds in the fatty acid portions. For instance, levels of a phosphatidylcholine from PC(34:1) to PC(34:3) may be determined.

In another embodiment, the PC comprises 36 carbon atoms in the fatty acid chains, and preferably 1 to 3 double bonds in the fatty acid portions. For instance, levels of a phosphatidylcholine from PC(36:1) to PC(36:3) may be determined.

In another embodiment, the PC comprises 38 carbon atoms in the fatty acid chains, and preferably 2 to 4 double bonds in the fatty acid portions. For instance, levels of a phosphatidylcholine from PC(38:2) to PC(38:4) may be determined.

In another embodiment, the PC comprises 40 carbon atoms in the fatty acid chains, and preferably 2 to 4 double bonds in the fatty acid portions. For instance, levels of a phosphatidylcholine from PC(40:2) to PC(40:4) may be determined.

In accordance with the claimed invention the phosphatidylcholine to be determined is selected from the group consisting of PC(32:0), PC(32:1), PC(34:1), PC(34:3), PC(36:1), PC(36:3), PC(38:2), PC(38:3), PC(38:4), PC(40:2), PC(40:3) and PC(40:4).

### Combinations of biomarkers

In general the method of the present invention may involve determining the level of any combination of two or more of the lipid species recited in the amended claims.

In a particularly preferred embodiment, the method comprises determining a level of TAG(54:2), CE(16:1) and PE(36:5), and optionally a phosphatidylcholine from PC 32:0 to PC 40:4.

### Comparison to control

The present method further comprises a step of comparing the level of the individual lipid species in the test sample to one or more control values. Typically a specific control value for each individual lipid species determined in the method is used. The control value may be a normal level of that lipid species, e.g. a level of the lipid in the same sample type (e.g. serum or plasma) in a subject who is not suffering from CVD. The control value may, for example, be based on a mean or median level of the lipid species in a control population of subjects, e.g. 5, 10, 100, 1000 or more healthy subjects (who may either be age- and/or gender-matched or unmatched to the test subject) who show no symptoms of CVD. Preferably the level of the lipid in the test sample is increased by at least 1%, 5%, at least 10%, at least 20%, at least 30%, or at least 50% compared to the control value.

The control value may be determined using corresponding methods to the determination of lipid levels in the test sample, e.g. using one or more samples taken from normal (healthy) subjects. For instance, in some embodiments lipid levels in control samples may be determined in parallel assays to the test samples. Alternatively, in some embodiments control values for the levels of individual lipid species in a particular sample type (e.g. serum or plasma) may already be available, for instance from published studies. Thus in some embodiments, the control value may have been previously determined, or may be calculated or extrapolated, without having to perform a corresponding determination on a control sample with respect to each test sample obtained.

### Association of lipid levels to CVD risk

In general, an increased level of any of the above lipid species in the test sample compared to the control value is indicative of an increased risk of CVD, particularly an increased long-term risk of atherosclerotic plaque rupture and/or associated thrombosis, which may result in an acute ischemic event such as myocardial infarction or stroke. The overall risk of CVD may be assessed by determining a number of different lipid biomarkers as discussed above, and combining the results. For instance, subjects may be stratified into low, medium, high and/or very high risk groups according to the number of individual lipid species which are elevated relative to control and/or the degree to which they are elevated.

### Methods of treatment

In one aspect, the present invention provides a method for preventing or treating cardiovascular disease in a subject. In particular, the method may be used to prevent the occurrence of acute ischemic events such as myocardial infarction and ischemic stroke, for instance by reducing the likelihood of atherosclerotic plaque rupture or associated thrombosis. By preventing or treating CVD, it is intended to encompass reducing the risk of CVD, reducing the severity of CVD and/or reducing mortality resulting from CVD.

The method for preventing or treating CVD typically comprises a first step of determining CVD risk in the subject by a method as described above. Following the determination of CVD risk, an appropriate intervention (e.g. prophylactic and/or therapeutic treatment) strategy may be selected for the subject, based on assessed risk level.

Typically if the subject shows a low level of CVD risk, no intervention may be necessary. For instance, if the subject's risk level is at or below a threshold level, no pharmaceutical or nutritional therapy may be required. The threshold level may correspond, for example, to a normal or mean level of risk in the general population.

Alternatively, if the subject shows an elevated risk of CVD, e.g. an elevated risk of experiencing an acute ischemic event such as myocardial infarction or stroke, the method may comprise a further step of administering an anti-CVD therapy to the subject. The anti- CVD therapy may comprise any prophylactic, therapeutic, pharmaceutical or nutritional agent which is effective in reducing CVD risk. Preferably, the therapy comprises an anti- atherosclerotic therapy (i.e. a therapy which reduces of prevents the development of atherosclerosis), an anticoagulant (e.g. a coumarin or vitamin K antagonist such as warfarin, low-dose aspirin or heparin) or a thrombolytic therapy (e.g. streptokinase or tissue plasminogen activator).

In a preferred embodiment, the anti-CVD therapy comprises an agent which is capable of reducing levels of one or more of the lipid species which are increased in the subject. For instance, and in accordance with the claimed invention, the agent is a statin or another HMG CoA reductase inhibitor. Suitable statins include atorvastatin, cerivastatin, fluvastatin, fluvastatin XL, lovastatin, pitavastatin, pravastatin, rosuvastatin or simvastatin. In another preferred embodiment, the drug is niacin (nicotinic acid); a cholesterol absorption inhibitor, such as ezetimibe or SCH-48461; a cholesteryl ester transfer protein (CETP) inhibitor, such as torcetrapib, anacetrapib or JTT- 705; a bile acids sequestrant, such as colesevelam, cholestyramine and colestipol; or a fibrate, such as fenofibrate, gemfibrozil, clofibrate, and bezafibrate. In another embodiment, the anti-CVD therapy comprises a phytosterol or phytostanol.

An advantage of the present invention is that an anti-CVD therapy can be selected which is effective in reducing levels of the specific lipid species associated with CVD risk which are elevated in an individual subject. Typically, different anti-CVD therapies (e.g. individual statins) may have differing effects on the profiles of individual lipid species. For instance, some statins may be more effective at reducing levels of CE(14:0) to CE(16:1), whereas other statins may be more effective at reducing levels of cholesterol esters comprising longer chain and/or more unsaturated fatty acid residues. Moreover, individual patients may respond better (in terms of a reduction of CVD-risk associated lipid species) to different statins due to various factors such as genetic variability and lifestyle.

Thus in embodiments of the present invention, anti-CVD therapy may be personalized to the subject, such that CVD-risk associated lipid levels are monitored in conjunction with a specific therapy targeted to reducing those individual lipid species in the subject. For instance, the method may comprise a further step of (re-)determining lipid levels in the subject (i.e. after the initial administration of an anti-CVD therapy), in order to assess the effectiveness of the therapy in reducing CVD risk. If the subject shows a reduction in CVD risk after the initial treatment phase, the therapy may be continued to maintain the CVD risk at reduced levels.

However, if the subject fails to respond adequately to the initial treatment (e.g. shows no significant reduction in specific lipid levels and/or CVD risk), the subject may be switched to an alternative treatment. For example, if a subject responds poorly to an initial statin-based treatment regime, an alternative statin may be administered to the subject. This process may be repeated, including selecting different dosages of individual agents, until a reduction in CVD-risk associated lipid levels is achieved. Typically, the subject may be maintained on a particular therapy (e.g. a pharmaceutical agent such as a statin) for at least 1 week, 2 weeks, 1 month or 3 months before the determination of lipid levels is repeated. The method may also be used to monitor the effects of lifestyle changes (such as changes in diet, exercise levels, smoking, alcohol consumption and so on) on CVD-risk associated lipid levels, and to identify an combination of factors which is effective in reducing the risk of disease.

### Further methods for determining CVD risk based on fatty acid chain length

As demonstrated in the Examples below, in subjects with elevated CVD risk there is a general shift from longer to shorter chain fatty acid residues in certain plasma lipid species, particularly TAGs and CEs and to a lesser extent PEs and PCs, compared to normal control subjects (see Fig.s 7 and 8).

The fatty acid composition of lipids from the test sample may be compared with that of a control sample, and the mean fatty acid chain length determined. Determining the mean fatty acid chain length may include determining the total number of carbon atoms in fatty acid residues in the relevant lipid species. In general, a decrease in mean fatty acid chain length (i.e. a shift from longer chain fatty acid residues to shorter chain fatty acid residues) in lipids in the test sample compared to the control is indicative of increase CVD risk. In some cases, the ratio of longer to shorter chain fatty acid residues in the relevant lipid species may be determined.

In particular embodiments the lipids are triacylglycerols (TAGs) or cholesterol esters (CEs). In the case of TAGs, species comprising a total of 54 or fewer carbon atoms in the fatty acid residues of the molecule (i.e. TAGs comprising shorter chain fatty acid residues) are typically increased in subjects having increased CVD risk, and/or species comprising a total of 56 or more carbon atoms in the fatty acid residues of the molecule (i.e. TAGs comprising longer chain fatty acid residues) are typically decreased in subjects having increased CVD risk. In the case of CEs, species comprising a total of 20 or fewer carbon atoms in the fatty acid residue of the molecule may be increased, and/or species comprising a total of 22 or more carbon atoms in the fatty acid residue of the molecule may be decreased in subjects with elevated CVD risk compared to the control.

In the case of phosphatidylethanolamines (PEs), a decrease in mean fatty acid chain length (e.g. an increase in PEs comprising a total of 38 or fewer carbon atoms in the fatty acid residues of the molecule, and/or a decrease in PEs comprising a total of 40 or more carbon atoms in the fatty acid portions of the molecule) is indicative of increased CVD risk.

Where phosphatidylcholines (PCs) are analysed, a decrease in mean fatty acid chain length (e.g. an increase in PCs comprising a total of 40 or fewer carbon atoms in the fatty acid residues of the molecule, and/or a decrease in PCs comprising a total of 42 or more carbon atoms in the fatty acid portions of the molecule) is indicative of increased CVD risk.

In the case of lysophosphatidylcholines (LPCs) a shift from shorter to longer chain fatty acid chain lengths is associated with decreased CVD risk. Thus in embodiments where LPCs are analysed, an increase in mean fatty acid chain length in the LPCs in the sample from the subject compared to the control indicates a decreased risk of CVD in the subject. For instance, an increase in LPCs comprising a total of 22 or more carbon atoms in the fatty acid residue of the molecule, and/or a decrease in LPCs comprising a total of 20 or fewer carbon atoms in the fatty acid residue of the molecule, may be indicative of decreased CVD risk.

In general, the method for determining CVD risk based on fatty acid chain length may be performed in combination with the methods described above involving the determination of the levels of particular lipid species. For instance, each method may be performed using similar techniques (e.g. mass spectrometry), using similar samples and controls. Moreover, the methods are both predictive of the risk of the same conditions (e.g. myocardial infarction and/or ischemic stroke) and may be used in combination with the same methods for preventing or treating disease. Whether the method involves detecting elevated levels of particular lipid species or detecting a shift from longer chain to shorter chain fatty acid residues, each of the methods described herein relies on changes in fatty acid composition in particular plasma lipid species which are associated with increased CVD risk.

The invention will now be described by way of example only with respect to the following specific embodiments.

### EXAMPLES

### Example 1

There is a clear need for new biomarkers capable of identifying patients at risk of plaque destabilization and rupture¹. Among various pathological features of atherosclerotic plaques that define their propensity to rupture, the content and composition of plaque lipids deserve special consideration². Altered lipid metabolism and dyslipidemia in the context of inflammation and oxidative stress are driving forces in the transition of stable to unstable plaques. We have recently performed comparative lipidomics profiling of carotid endarterectomy specimens from symptomatic versus asymptomatic patients³ and highlighted the existence of a characteristic lipid signature within unstable human plaques. The present study further investigates molecular lipid profiles in the circulation.

While nuclear magnetic resonance spectroscopy is fast and cheap⁴, its possibilities to resolve individual lipid species are limited due to peak overlap⁵. In contrast, shotgun lipidomics using mass spectrometry (MS) can screen and simultaneously analyze hundreds of lipid species in non-separated lipid extracts⁶. Thus, MS is the preferred method for in-depth studies on lipid-related pathomechanisms in the manifestation of cardiovascular disease (CVD). Recent advances in MS allow the application of this technology to epidemiological cohorts^{7,8}. In this study, we performed lipidomics profiling in the prospective population-based Bruneck Study and analyzed the association of 135 distinct lipid species with CVD risk over a 10-year observation period.

### METHODS

### Study Subjects and Examination

The Bruneck Study is a prospective, population-based survey on the epidemiology and pathogenesis of atherosclerosis and cardiovascular disease⁹⁻¹². At the 1990 baseline evaluation, the study population comprised an age- and sex- stratified random sample of all inhabitants of Bruneck (125 men and 125 women from each of the fifth through eighth decades of age, all Caucasian). In 2000, 702 subjects were still alive and participated in the second quinquennial follow-up. Plasma samples for lipidomics analyses were available for 685 individuals (97.6%). During the follow-up between 2000 and 2010, detailed information about fatal and nonfatal new-onset CVD was carefully collected (follow-up, 100% complete). The study protocol was approved by the ethics committees of Bolzano and Verona, and conformed to the Declaration of Helsinki, and all study subjects gave their written informed consent. Risk factors were assessed by means of validated standard procedures as described previously⁹⁻¹³.

The composite CVD endpoint included incident fatal and non-fatal myocardial infarction, ischemic stroke, as well as sudden cardiac death. Presence of myocardial infarction was assessed by World Health Organization criteria¹⁴ while ischemic stroke was classified according to the criteria of the National Survey of Stroke¹⁵. Ascertainment of events did not rely on hospital discharge codes or the patient's self-report but on a careful review of medical records provided by the general practitioners, death certificates and Bruneck Hospital files. A major advantage of the Bruneck Study is that virtually all inhabitants of Bruneck are referred to the local hospital, which closely works together with the general practitioners. This allows retrieval of full medical information.

### Laboratory Methods

As part of the 2000 follow-up, citrate plasma samples were drawn after an overnight fast and 12 hours of abstinence from smoking. Plasma samples were aliquoted and immediately stored at -80°C. Total cholesterol, high-density lipoprotein (HDL) cholesterol and triglyceride content were measured by standard procedures. Low-density lipoprotein (LDL) was calculated by the Friedewald formula¹⁶, except in patients with triglycerides higher than 400 mg/dL, in whom LDL was also measured directly.

### Lipid Extraction

Lipid extraction¹⁷ and analysis was done in batches of 24 samples including one quality control in random order. 10 µL plasma was mixed in glass vials with 10 µL 0.15 M NaCl, 100 µL CHCl3/MeOH 2:1 and 10 µL internal standard containing 75.2 pmol LPC(19:0), 500.7 pmol PC(17:0/17:0), 75.3 pmol SM(d18:1/12:0), 1.0 pmol LPE(17:1), 3.8 pmol PE(15:0/15:0), 18.3 pmol PS(17:0/17:0), 10.0 pmol LPS(17:1), 257.6 pmol CE(17:0) (all from Avanti Polar Lipids) and 102.1 pmol TAG(17:0/17:0/17:0) (from Sigma) per µL plasma in CHCl3/MeOH 2:1. After incubation at room temperature for 30 minutes, samples were centrifuged at 10 000 rpm for 3 minutes and an aliquot of 50 µL of the lower layer was transferred into a new glass vial.

### Mass Spectrometry (MS)

For MS analysis³, 10 µL of lipid extract was diluted with 90 µL CHCl3/MeOH/Isopropanol 1:2:4 containing 7.5 mM NH4OAc and 10 µM LiOH. Just before MS analysis, samples were centrifuged at 12 000 rpm for 2 minutes and 25µL were transferred into a Twintec plate (Eppendorf). 10 µL extract was analyzed by direct infusion with a QqQ-MS (TSQ Vantage, Thermo Fisher Scientific) equipped with a TriVersa NanoMate (Advion BioSciences) and controlled by Chipsoft software version 8.1.0.928 (Advion BioSciences). An ionization voltage of 0.95 to 1.40 kV and a gas pressure of 1.25 psi were used (1). Spectra were automatically acquired with rolling scan events by a sequence subroutine operated under Xcalibur software version 2.0.7 (Thermo Fisher Scientific). Within the first minute, full MS spectra were acquired in positive mode followed by 4 minutes of rolling scan events with different lipid class specific neutral loss (NL) and precursor ion (PI) scans 3. For NL and PI scans argon with a pressure of 1.0 mTorr was used as a collision gas (NL 141.0@25 for LPE/PE, NL 185.0@22 for LPS/PS, NL213.0@52 for SM, PI 184.1@35 for LPC/PC, PI 369.3@20 for CE (2-4). A total of 18 PI scans and NL scans for different lipid classes plus additional scans for fatty acids resulted in the detection of 138 lipid species attributable to the eight different lipid classes. Of these, the three lipid species PC(22:0), PC(24:0) and PC(24:1) were excluded because of very low concentrations (more than 95% zero values) leaving 135 for the main analysis. To calculate the amount of the different lipid species, authentic standards were spiked into the samples before extraction. Results for the various lipid species are expressed as pmol per µL plasma.

### Statistical Analysis

Cox proportional hazards models were built to assess the association of each individual lipid species with CVD risk. The proportional hazards assumption was tested by computing the significance level of the correlation coefficient between Kaplan-Meier-transformed survival time and scaled Schoenfeld residuals for all variables in all models. Adjustment for multiple testing was performed by means of the Benjamini-Hochberg procedure which is more appropriate in an "-omics" setting than the Bonferroni correction¹⁹. Results were very similar after log-transforming the concentrations of lipid species. For ease of interpretation, only results derived from untransformed data are presented. Potential undue influence of outliers was examined by re-running analyses after exclusion of cases with lipid concentrations more than three standard deviations higher or lower than the mean. This approach again produced almost identical results. For comparison purposes we calculated the sum of all individual lipid species within each lipid class and the respective first principal components.

In the network inference analysis, neighborhoods of interconnected lipids were defined using Topological Overlap Measure (TOM²⁰) - a mathematical metric of putative functional similarity. TOM is proportional to the number of neighbors that a pair of lipids has in common and two nodes having a higher topological overlap are more likely to belong to the same functional class²¹. The lipid species were clustered using a weighted co-expression network to determine modules of highly interconnected lipids²². The modules are described by the first principal component of the species present in the module and were correlated with age, sex, HDL cholesterol, LDL cholesterol, total cholesterol, and triglyceride levels.

For variable selection in the setting of high data dimensionality and extensive inter-correlations, L1-regularized Cox regression was used, which implements the least absolute shrinkage and selection operator (LASSO) algorithm²³. This model considered all lipid species simultaneously and age, sex and statin use were included as unpenalized explanatory variables. The optimal hyper-parameter λ was chosen as the one maximizing the partial likelihood as determined by ten-fold cross-validation. To account for the potentially large variance of cross-validation, this process was repeated 1000 times with different data partitions. Two analyses with alternative selection procedures were run for validation purposes. Multivariable effect sizes and confidence intervals for the final LASSO selection of lipid species were calculated by conventional unpenalized Cox regression to facilitate traditional formal inference.

The incremental predictive value of selected lipid species for CVD risk prediction was assessed by measures of risk discrimination (changes in Harrell's C- index²⁴) and risk reclassification (categorical²⁵), continuous and prospective net reclassification indices²⁶ [NRI] and integrated discrimination improvement²⁵ [IDI]).

The categorical NRIs were calculated based on 10-year risk categories <5%, 5-10%, 10-20%, and >20%. We evaluated the improvement in CVD risk prediction when selected lipid species were used (1) in addition to conventional risk factors (age, sex, diabetes, smoking status, systolic blood pressure, total cholesterol and HDL cholesterol) and (2) in replacement of standard lipid components in the Framingham Risk Score. Analyses were performed using R 2.15.1 and STATA 12; graphics were created with the ggplot2 package²⁷.

### RESULTS

### Molecular Lipid Profiling in the Bruneck Study

Over a follow-up period of 10 years (5902 person-years), 90 participants experienced a CVD event, corresponding to an incidence rate of 15.2 (95% confidence interval (CI), 12.4 to 18.7) per 1000 person-years. Shotgun lipidomics detected 135 lipid species attributable to eight different lipid classes: phosphatidylcholine (PC), lysophosphatidylcholine (LPC), cholesterol ester (CE), sphingomyelin (SM), phosphatidylserine (PS), phosphatidylethanolamine (PE), lysophosphatidyl-ethanolamine (LPE), and triacylglycerol (TAG). Figure 1 shows significance levels for the associations of individual lipid species (dots) and two lipid class summary measures (bars) with incident CVD (logarithmic y-axis). A number of individual lipid species within the classes of TAG, CE, LPC, and PC outperformed the respective summary measures.

### Associations of Lipid Species with Cardiovascular Risk

Considering each lipid species separately, 50 members of TAGs, CEs, PEs, PCs, LPCs, and SMs were significantly associated with CVD risk (**Table 1**). When controlling for multiple comparisons, 28 lipids maintained significance. In **Figure 2****,** all 135 lipid species are plotted as circles with their position in the two-dimensional lipid class graphs determined by the total acyl chain carbon numbers (x-axis) and double bond content (y-axis). The shade of each circle depicts the strength and direction (positive or negative) of given associations. The size of the circle indicates the level of statistical significance. Of note, associations were most pronounced for TAGs and CEs of lower carbon number and double bond content (i.e. saturated and mono-unsaturated fatty acids) and the risk profile was complemented by PE/PCs, SMs (both positive) and LPCs (negative). Subgroup analyses were performed in men and women and age strata. These analyses did not yield evidence of differential effects (the number of interaction terms with a P value <0.05 was lower than that expected by chance: 2 and 3 versus 7) and justify pooled analysis of sexes and of the entire age range. As expected, statin therapy had some effect on the association patterns. Thus, analyses were adjusted for statin use and key computations were repeated after exclusion of participants on statins.

**Lipidomics Signature for Cardiovascular Risk**To identify a lipidomics signature of CVD risk, we applied LASSO and two alternative selection algorithms and network inference as backup methods. In L1-regularized Cox regression analysis, the three lipid species TAG(54:2), CE(16:1), and PE(36:5) were most consistently related to incident CVD (**Figure 3**). Details on inclusion fractions in 1000 runs are provided in **Table 1** and compared with models employing backward selection and best subset algorithms. Multivariable effect sizes and confidence intervals for the LASSO selection of lipid species are shown in **Figure 4****.** After exclusion of 65 subjects taking statins, LASSO still selected TAG(54:2) and CE(16:1) but gave preference to PC(32:1) over PE(36:5) within the PE/PC cluster.

### Lipid Network Analysis

Differences in these three lipid species - TAG(54:2), CE(16:1) and PE(36:5) - were reflected in the modules of the network inference analysis (**Fig**.**5**). As expected, the module containing all TAG species was strongly correlated with total triglyceride levels (Pearson correlation coefficient 0.86). Similarly, total and LDL cholesterol showed strong correlations with the modules containing the bulk of CE and PC/PE species (Pearson correlation coefficients 0.38 - 0.55). The connectivity within and between modules was calculated for each lipid and the TAGs show a high intra-connectivity and a low inter-connectivity compared with the other lipids and modules. TAG_54_2 had one of the largest differences (1.83) between the intra- and inter- connectivity between the modules, whereas CE_16_1 had one of the lowest differences (-0.25) and PE_36_5 was between (0.03). PE_36_5 and TAG_54_2 both had a high proportion of connections (83.3% and 61.0%) that were associated with CVD whereas CE_16_1 had a lower proportion (30.1%) associated with CVD (P=0.02).

### CVD Risk Prediction and Classification

Addition of TAG(54:2), CE(16:1), and PE(36:5) to a model including conventional risk factors increased the C-index by 0.0249 (95%Cl, 0.0055 to 0.0443; P=0.012) and yielded an integrated discrimination improvement of 0.024 (95%Cl, 0.006 to 0.041). Corresponding data for the addition of six lipid species were 0.0465 (95%Cl, 0.0178 to 0.0752) and 0.0460 (95%Cl, 0.0194 to 0.0727). Net reclassification improvements were calculated for the 10-year risk categories <5%, 5 to 10%, 10% to 20%, and >20% and are summarized in **Figure 6****.** In brief, consideration of TAG(54:2), CE(16:1), and PE(36:5) on top of conventional risk factors resulted in a significant improvement in risk stratification (NRI=9.53%, P=0.05) that was mainly driven by correct reclassification of non-cases (NRI=10.64%, P<0.001). Similarly, replacement of standard lipid measures of the Framingham Risk Score (total and HDL cholesterol) with TAG(54:2), CE(16:1), and PE(36:5) resulted in a significant improvement in risk discrimination and 10-year risk reclassification (**Figure 6**). Findings were similar after exclusion of subjects with prior CVD.

In the Bruneck study, there was a clear shift in the number of carbon atoms for long chain fatty acids that are associated with cardiovascular risk, in both CE as well as TAG (**Figure 7**). Based at the different combinations of fatty acids, which could combine to create the TAGs with statistical significance in the Bruneck cohort (TAG 50:1, TAG 50:2, TAG 50:3, TAG 52:2, TAG 52:3, TAG 52:5, TAG 54:2, TAG 56:1, TAG 56:5 and TAG 56:6), there was a high frequency of myristate (14:0), palmitate (16:0) and oleate (18:1). Similarly, for CE, the highest risk in the Bruneck study was associated with CE containing palmitoleate (16:1) but significant associations were also obtained for myristate (14:0), palmitate (16:0), and oleate (18:1). Since TAG and CE constitute major lipid species, changes in their fatty acid composition may be representative of the overall fatty acid pool in the circulation.

### DISCUSSION

This is the first prospective population-based study reporting a systematic analysis of the plasma lipidome in the context of CVD. It allows four main conclusions:(1) There is a broad diversity of potential cardiovascular effects of lipid species within most lipid classes, and as a consequence, individual lipids outperform lipid summary measures with regards to CVD risk prediction (**Fig**. **1**). (2) TAGs of low carbon number and double bond content show the strongest and most consistent associations with CVD, surpassing CEs and PE/PCs (**Fig**. **2**). (3) Molecular lipid profiling by MS results in a significant improvement in CVD risk discrimination and classification beyond the information provided by classic risk factors including conventional lipid measures (**Fig**. **6**). (4) The stronger association of certain lipid species with CVD can, at least in part, be explained by a shift in the plasma fatty acid composition.).

### Post-genomics Technologies

Recent advances in post-genomics technologies allow the interrogation of CVD at the transcriptome (RNA), proteome (proteins), and metabolome (small molecules) level. Previous metabolomic studies have suggested significant associations between CVD and branched chain amino acids, acetylcarnitines, free fatty acids^{28,29} and metabolites linked to choline metabolism³⁰. We assessed 135 lipid species in a prospective population-based cohort using shotgun lipidomics. Lipids are among the main culprits in vessel pathology and thus represent a prime target for metabolomic profiling in cardiovascular research. Indeed, several lipid species within six out of eight lipid classes - TAG, CE, PE, PC, SM, and LPC - showed significant associations with future CVD. Importantly, lipidomics interrogated information that was not captured by the established CVD risk factors based on the conventional biochemical measurements of triglycerides, total cholesterol, HDL and LDL cholesterol only.

### Triglyceride Species and CVD

A specific cluster of TAGs with low carbon number and double bond content, i.e. saturated and mono-unsaturated acyl chains was most consistently associated with CVD (**Figures 2** **and** **3****,** lowest P value 4.6x10⁻⁷). This observation suggests that the relevance of TAGs in the context of both diseases may have been underestimated in previous research by an unwarranted focus on total triglycerides. Large meta-analyses have yielded solid evidence of an association between triglycerides and CVD³², but it remains controversial whether triglycerides levels are just a marker of pro-atherogenic lipoprotein dynamics and composition^{33,34}, or causally related to lipoprotein retention in the vessel wall, plaque stability and thrombogenicity^{34,35}. Our finding that certain TAG species rather than total triglycerides confer increased CVD risk supports the latter view.

### CE Species and CVD

CEs showed significant associations with incident CVD. Similar to TAGs, the most strongly associated CEs had a low carbon number and double bond content (**Figure 2**). CE(16:1) is derived from acyl-CoA:cholesterol acyl transferase-2 (ACAT2) activity. Interestingly, lipid profiling in transgenic mice identified palmitoleate as an adipose tissue-derived lipid hormone that can modulate systemic insulin sensitivity³⁹. It is also one of the main products in the endogenous synthesis of non-essential fatty acids⁴⁰: The initial major product is palmitate (16:0), which can be processed to palmitoleate (16:1). Myristate (14:0) is another possible minor product of fatty acid synthesis. Both, CE(14:0) and CE(16:0) showed significant, albeit weaker, associations with incident CVD in the Bruneck study.

### Other Lipid Species and CVD

Besides CEs and TAGs, several PE/PC species and few SMs were linked to CVD risk. For example, PC38:3 showed by far the strongest association with CVD in the Bruneck study (**Figure 2**). Similarly, SM(34:2) was positively associated with CVD, which is consistent with the observed shift in the fatty acid composition in other lipid classes, ie. TAG and CE. In contrast, LPCs showed an inverse relationship. The latter finding is counterintuitive because pro-atherogenic lipoprotein-associated phospholipase A2 (Lp-PLA2) activity results in the generation of LPCs and LPC content is elevated in plaques³. However, it is in agreement with the findings of a recent study on coronary artery disease (CAD) and may reflect increased LPC catabolism and clearance from the circulation³⁹. Moreover, circulating LPC is partially generated by lecithin- cholesterol acyltransferase and low lecithin-cholesterol acyltransferase activity has been linked to CAD^{40,41}.

### Molecular Lipid Signature and CVD

Within the system-wide lipid network, three lipid species were most informative for CVD risk in our study: TAG(54:2), CE(16:1), and PE(36:5) (**Figures 3** and **4****, Table 1**). Addition of these lipid species to models containing standard CVD risk factors improved risk discrimination and stratification and the same was true when standard lipid measures were replaced by these three variables (**Figure 6**). Five-fold cross validation (300 repetitions) in the Bruneck cohort yielded evidence that the estimates of predictive accuracy obtained in the entire Bruneck population are robust for the top choice of three lipid species.

### Shotgun vs targeted lipidomics techniques

Shotgun lipidomics offers a comprehensive overview⁴³, but not full coverage of the plasma lipidome. One drawback of the technique is measurement variability, reflected in higher coefficients of variation, particularly for low-abundant lipid species like LPCs. This, however, would be expected to weaken evident associations rather than to create spurious ones ('regression dilution bias').

The alternative method of targeted lipidomics with chromatographic separation and multiple reaction monitoring allows more accurate quantitation of less abundant lipid species, but is best suited for assessing a limited number of lipid species within a single run due to the narrow time window of eluting peaks. Targeted lipidomics also necessitates standards for each lipid species in order to minimize errors arising from changes in lipid ionization efficiency and variations due to chromatography. Memory effects on the column are another concern.

In contrast, shotgun lipidomics simultaneously screens and analyses lipid species in non-separated lipid extracts^{44,45}. It uses class-specific standards. Thus, it does not require *a priori* decisions on which lipid species to measure. The set-up for shotgun lipidomics also excludes variation by chromatography and minimizes sample cross-contamination in a high-throughput setting. Strengths of our study include its size, prospective design, thorough characterization of study subjects, complete and high-quality assessment of outcome events, and consistency of findings in sensitivity analyses.

### Summary

Our findings challenge the current practice of lipid management with its main focus on cholesterol and on lipid levels (best addressed by drug therapy) rather than lipid composition (potentially targetable by dietary measures⁴⁶). Lipidomics offers unprecedented opportunities to move beyond the genome to address the clinical heterogeneity of CVD, and to provide therapies based on targeting individual lipid species associated with CVD risk.

### Example 2

In this Example, samples from the TwinsUK Cohort (n=1453, 45 CVD cases) were used for validation purposes.

**Study population.** The TwinsUK study, started in 1992, encompasses the biggest adult twin registry in the UK with about 12,000 twins studied for the genetic and environmental etiology of age-related complex traits⁴⁷. The cohort has extensive demographic, physiological, behavioral and lifestyle data available and is one of the most deeply phenotyped and genotyped cohorts in the world. Details about the study procedures and advances have been documented in our recent cohort profile⁴⁸.

**Metabolomic assessment in TwinsUK study.** Metabolomic profiles of 6,056 twins have been assessed using a non-targeted platform (Metabolon Inc., Durham, USA) on plasma samples. The Metabolon platform incorporates two separate ultra-high performance liquid chromatography/tandem mass spectrometry (UHPLC/MS/MS) injections, optimised for basic and acidic species, and one gas chromatography/mass spectrometry (GC/MS) injection per sample⁴⁹. The platform has detected and quantified concentration of 510 metabolites in our study population (299 known and 211 unknown molecules) including 70 amino-acids, 122 lipids, 13 carbohydrates, 11 vitamins, 11 nucleotides, 21 peptides, 45 xenobiotics and 6 energy metabolites. Out of 6,056 twins with available profiles, 5,621 (93%) are female and the majority (n=4,668) are >40 years of age (1,190 are >65 years) at the time of metabolomic measurements. Only unrelated participants were included in the present analysis of plasma free FA following methanol precipitation. Except pentadecanoate (15:0), which was measured by GC-MS, all FA were measured by LC-MS/MS in negative ion mode.

### Comparison with the TwinsUK Cohort

In the Bruneck Study, there was a clear shift in the chain length of FA among lipid species associated with CVD risk, especially for CE, SM and TAG. These three classes accounted for the majority of plasma lipids and thus dominated the FA content in complex lipids (**Figure 9A**). Based on the different combinations of FA, which could combine to create the TAGs with statistical significance in the Bruneck cohort (TAG 50:1, TAG 50:2, TAG 50:3, TAG 52:2, TAG 52:3, TAG 52:5, TAG 54:2, TAG 56:1, TAG 56:5 and TAG 56:6), there was a high frequency of myristate (14:0), palmitate (16:0), stearate (18:0), myristoleate (14:1), palmitoleate (16:1), and oleate (18:1). Similarly, in CEs and SMs related to CVD risk in the Bruneck Study the most abundant FA were myristate (14:0), palmitate (16:0), palmitoleate (16:1) and oleate (18:1) (**Figure 9A**). For replication, we determined the free FA composition in plasma samples of the TwinsUK Cohort by MS (**Figure 9B**). In close agreement with the data from the Bruneck study, levels of myristate (14:0), palmitate (16:0), palmitoleate (16:1) and oleate (18:1) were more strongly associated with CVD than other FA.

### REFERENCES

1. Gerszten RE, Wang TJ. The search for new cardiovascular biomarkers. Nature 2008;451:949-952
2. Didangelos A, Stegemann C, Mayr M. The -omicsera: Proteomics and lipidomics in vascular research. Atherosclerosis 2012;221:12-17
3. Stegemann C, Drozdov I, Shalhoub J, Humphries J, Ladroue C, Didangelos A, Baumert M, Allen M, Davies AH, Monaco C, Smith A, Xu Q, Mayr M. Comparative lipidomics profiling of human atherosclerotic plaques. Circ. Cardiovasc. Genet. 2011;4:232-242
4. Mayr M. Metabolomics: Ready for the prime time? Circ. Cardiovasc. Genet. 2008;1:58-65
5. Kettunen J, Tukiainen T, Sarin AP, Ortega-Alonso A, Tikkanen E, Lyytikainen LP, Kangas AJ, Soininen P, Wurtz P, Silander K, Dick DM, Rose RJ, Savolainen MJ, ViikariJ,KahonenM,LehtimakiT,PietilainenKH,InouyeM,McCarthyMI,JulaA, Eriksson J, Raitakari OT, Salomaa V, Kaprio J, Jarvelin MR, Peltonen L, Perola M, Freimer NB, Ala-Korpela M, Palotie A, Ripatti S. Genome-wide association study identifies multiple loci influencing human serum metabolite levels. Nat. Genet. 2012;44:269-276
6. Sun G, Yang K, Zhao Z, Guan S, Han X, Gross RW. Shotgun metabolomics approach for the analysis of negatively charged water -soluble cellular metabolites from mouse heart tissue. Anal. Chem.2007;79:6629-6640
7. Shah SH, Kraus WE, Newgard CB. Metabolomic profiling for the identification of novel biomarkers and mechanisms related to common cardiovascular diseases: Formandfunction.Circulation.2012;126:1110-1120
8. Graessler J, Schwudke D, Schwarz PE, Herzog R, Shevchenko A, Bornstein SR. Top-down lipidomics reveals ether lipid deficiency in blood plasma of hypertensive patients. PLoS. ONE.2009;4:e6261
9. Zampetaki A, Willeit P, Tilling L, Drozdov I, Prokopi M, Renard JM, Mayr A, Weger S, Schett G, Shah A, Boulanger CM, Willeit J, Chowienczyk PJ, Kiechl S, Mayr M. Prospective study on circulating micrornas and risk of myocardial infarction. J. Am. Coll. Cardiol. 2012;60:290-299
10. Kiechl S, Lorenz E, Reindl M, Wiedermann CJ, Oberhollenzer F, Bonora E, Willeit J, Schwartz DA. Toll-like receptor 4 polymorphisms and atherogenesis. N. Engl. J. Med.2002;347:185-192
11. Kiechl S, Schett G, Schwaiger J, Seppi K, Eder P, Egger G, Santer P, Mayr A, Xu Q, Willeit J. Soluble receptor activator of nuclear factor-kappa b ligand and risk for cardiovasculardisease. Circulation 2007;116:385-391
12. Zampetaki A,Kiechl S, Drozdov I, Willeit P, Mayr U, Prokopi M, Mayr A, Weger S, Oberhollenzer F, Bonora E, Shah A, Willeit J, Mayr M. Plasma microrna profiling reveals loss of endothelial mir-126 and other micrornas in type 2 diabetes. Circ. Res. 2010;107:810-817
13. Willeit P, Willeit J, Mayr A, Weger S, Oberhollenzer F, Brandstatter A, Kronenberg F, Kiechl S. Telomere length and risk of incident cancer and cancer mortality. Jama.2010;304:69-75
14. Organization WH. Report of the fifth working group. IHD. Register.1971
15. Walker AE, Robins M, Weinfeld FD. The national survey of stroke. Clinical findings.*Stroke*.1981;12:I13-44
16. Friedewald WT, Levy RI, Fredrickson DS. Estimation of the concentration of low-density lipoprotein cholesterol in plasma, without use of the preparative ultracentrifuge. Clin. Chem.1972;18:499-502
17. Folch J, Lees M, Sloane Stanley GH. A simple method for the isolation and purification of total lipids from animal tissues.J. Biol. Chem.1957;226:497-509
18. Haimi P, Uphoff A, Hermansson M, Somerharju P. Software tools for analysis of mass spectrometric lipidome data. Anal. Chem.2006;78:8324-8331
19. Benjamini Y, Hochberg Y. Controlling the false discovery rate: A practical and powerfull approach to multiple testing.J.. R. Statistic. Soc.1995;57:289-300
20. Yip AM, Horvath S. Gene network interconnectedness and the generalized topological overlap measure. BMC. Bioinformatics.2007;8:22
21. Ravasz E, Somera AL, Mongru DA, Oltvai ZN, Barabasi AL. Hierarchical organization of modularity in metabolic networks.Science.2002;297:1551-1555
22. Langfelder P, Horvath S. Wgcna: An r package for weighted correlation network analysis. BMC. Bioinformatics.2008;9:559
23. Goeman JJ. L1 penalized estimation in the cox proportional hazards model. Biometrical. journal. Biometrische. Zeitschrift.2010;52:70-84
24. Harrell FE, Jr., Lee KL, Mark DB. Multivariable prognostic models: Issues in developing models, evaluating assumptions and adequacy, and measuring and reducing errors. Stat. Med.1996;15:361-387
25. Pencina MJ, D'Agostino RB, Sr., D'Agostino RB, Jr., Vasan RS. Evaluating the added predictive ability of a new marker: From area under the roc curve to reclassification and beyond. Stat. Med.2008; 27:157-172; discussion207-112
26. Pencina MJ, D'Agostino RB, Sr., Steyerberg EW. Extensions of net reclassification improvement calculations to measure usefulness of new biomarkers. Stat. Med. 2011;30:11-21
27. Wickham H. Ggplot2:. Elegant graphics for data analysis. New York: Springer 2009.
28. Shah SH, Sun JL, Stevens RD, Bain JR, Muehlbauer MJ, Pieper KS, Haynes C, Hauser ER, Kraus WE, Granger CB, Newgard CB, Califf RM, Newby LK. Baseline metabolomic profiles predict cardiovascular events in patients at risk for coronary artery disease. Am. Heart. J.2012;163:844-850e841
29. Shah SH, Hauser ER, Bain JR, Muehlbauer MJ, Haynes C, Stevens RD, WennerBR, Dowdy ZE, Granger CB, Ginsburg GS, Newgard CB, Kraus WE. High heritability of metabolomic profiles in families burdened with premature cardiovascular disease. Mol. Syst. Biol. 2009;5:258
30. Wang Z, Klipfell E, Bennett BJ, Koeth R, Levison BS, Dugar B, Feldstein AE, Britt EB, Fu X, Chung YM, Wu Y, Schauer P, Smith JD, Allayee H, Tang WH, DiDonato JA, Lusis AJ, Hazen SL. Gut flora metabolism of phosphatidylcholine promotes cardiovascular disease. Nature 2011;472:57-63
31. Rhee EP,Cheng S,Larson MG, Walford GA, Lewis GD, McCabe E, Yang E, Farrell L, Fox CS, O'Donnell CJ, Carr SA, Vasan RS, Florez JC, Clish CB, Wang TJ, Gerszten RE. Lipid profiling identifies a triacylglycerol signature of insulin resistance and improves diabetes prediction in humans. J. Clin. Invest. 2011;121:1402-1411
32. Di Angelantonio E, Sarwar N, Perry P, Kaptoge S, Ray KK, Thompson A, Wood AM, Lewington S, Sattar N, Packard CJ, Collins R, Thompson SG, Danesh J. Major lipids, apolipoproteins, and risk of vascular disease. Jama.2009;302:1993-2000
33. Miller M, Stone NJ, Ballantyne C, Bittner V, Criqui MH, Ginsberg HN, Goldberg AC, Howard WJ, Jacobson MS, Kris-Etherton PM, Lennie TA, Levi M, Mazzone T, Pennathur S. Triglycerides and cardiovascular disease: A scientific statement fromtheamericanheartassociation.Circulation.2011;123:2292-2333
34. Chapman MJ, Ginsberg HN, Amarenco P, Andreotti F, Boren J, Catapano AL, Descamps OS, Fisher E, Kovanen PT, Kuivenhoven JA, Lesnik P, Masana L, Nordestgaard BG, Ray KK, Reiner Z, Taskinen MR, Tokgozoglu L, Tybjaerg K, Hansen A, Watts GF. Triglyceride-rich lipoproteins and high-density lipoprotein cholesterol in patients at high risk of cardiovascular disease: Evidence and guidance for management. Eur. Heart. J.2011;32:1345-1361
35. Sambola A, Osende J, Hathcock J, Degen M, Nemerson Y, Fuster V, Crandall J, Badimon JJ. Role of risk factors in the modulation of tissue factor activity and blood thrombogenicity. Circulation.2003;107:973-977
36. Sarwar N, Sandhu MS, Ricketts SL, Butterworth AS, Di Angelantonio E, Boekholdt SM, Ouwehand W, Watkins H, Samani NJ, Saleheen D, Lawlor D, Reilly MP, Hingorani AD, Talmud PJ, Danesh J. Triglyceride-mediated pathways and coronary disease: Collaborative analysis of 101 studies.Lancet.2010;375:1634-1639
37. Miller CD, Thomas MJ, Hiestand B, Samuel MP, Wilson MD, Sawyer J, Rudel LL. Cholesteryl esters associated with acyl-coa:Cholesterol acyltransferase predict coronary artery disease in patients with symptoms of acute coronary syndrome. Academic emergency medicine: official journal of the Society for Academic Emergency Medicine 2012;19:673-682
38. Ohrvall M, Berglund L, Salminen I, Lithell H, Aro A, Vessby B. The serum cholesterol ester fatty acid composition but not the serum concentration of alpha tocopherol predicts the development of myocardial infarction in 50-year-oldmen: 19 years follow-up. Atherosclerosis.1996;127:65-71
39. Croset M, Brossard N, Polette A, Lagarde M. Characterization of plasma unsaturated lysophosphatidylcholines in human and rat. Biochem. J. 2000;345Pt 1:61-67
40. Rasmiena AA, Ng TW, Meikle PJ.Metabolomics and ischaemic heart disease. Clin. Sci. (Lond) 2013;124:289-306
41. Duivenvoorden R, Holleboom AG, van den Bogaard B, Nederveen AJ, de Groot E, Hutten BA, Schimmel AW, Hovingh GK, Kastelein JJ, Kuivenhoven JA, Stroes ES. Carriers of lecithin cholesterol acyltransferase gene mutations have accelerated atherogenesis as assessed by carotid 3.0-t magnetic resonance imaging [corrected].J. Am. Coll. Cardiol.2011;58:2481-2487
42. Meikle PJ, Wong G, Tsorotes D, Barlow CK, Weir JM, Christopher MJ, Macintosh GL, Goudey B, Stern L, Kowalczyk A, Haviv I, White AJ, Dart AM, Duffy SJ, Jennings GL, Kingwell BA. Plasma lipidomic analysis of stable and unstable coronary artery disease. Arterioscler. Thromb. Vasc. Biol.2011;31:2723-2732
43. Han X, Gross RW. Global analyses of cellular lipidomes directly from crude extracts of biological samples by esi mass spectrometry: A bridge to lipidomics. J. Lipid. Res.2003;44:1071-1079
44. Han X, Gross RW. Quantitative analysis and molecular species fingerprinting of triacylglyceride molecular species directly from lipid extracts of biological samples by electrospray ionization tandem mass spectrometry. Anal. Biochem. 2001;295:88-100
45. Gross RW, Han X. Shotgun lipidomics of neutral lipids as an enabling technology for elucidation of lipid-related diseases. Am. J. Physiol. Endocrinol. Metab. 2009;297:E297-303
46. Castro-Perez JM, Roddy TP, Shah V, McLaren DG, Wang SP, Jensen K, Vreeken RJ, Hankemeier T, Johns DG, Previs SF, Hubbard BK. Identifying static and kinetic lipid phenotypes by high resolution uplc-ms: Unraveling diet-induced changes in lipid homeostasis by coupling metabolomics and fluxomics. J. Proteome. Res. 2011;10:4281-4290.
47. Spector TD, Williams FM. The UK Adult Twin Registry (TwinsUK). Twin Res Hum Genet. 2006;9:899-906.
48. Moayyeri A, Hammond CJ, Valdes AM, Spector TD. Cohort Profile: TwinsUK and Healthy Ageing Twin Study. Int J Epidemiol. 2013;42:76-85.
49. Evans AM, DeHaven CD, Barrett T, Mitchell M, Milgram E. Integrated, nontargeted ultrahigh performance liquid chromatography/electrospray ionization tandem mass spectrometry platform for the identification and relative quantification of the small molecule complement of biological systems. Anal Chem. 2009;81:6656-6667.

**Table 1. Inclusion fractions of the ten individual lipid species most frequently selected in three different selection procedures.**

| **LASSO Selected[%]** | **Variable** | **BESTSUBSET Selected[%]** | **Variable** | **STEPWISE Selected[%]** | **Variable** |
|---|---|---|---|---|---|
| **CE(16:1)** | **100.0** | **TAG(54:2)** | 99.7 | **PE(36:5)** | 72.4 |
| **TAG(54:2)** | **100.0** | **CE(16:1)** | 43.7 | **TAG(54:2)** | 69.5 |
| **PE(36:5)** | **95.3** | PC(O.34:1) | 35.1 | TAG(50:3) | 68.2 |
| SM(34:2) | 51.9 | LPC(18:1) | 34.7 | TAG(52:5) | 66.3 |
| LPC(20:5) | 25.3 | **PE(36:5)** | 28.3 | TAG(52:2) | 62.6 |
| LPC(22:6) | 19.6 | TAG(54:3) | 26.1 | **CE(16:1)** | 56.0 |
| PE(38:3) | 4.2 | PS(38:4) | 23.5 | PE(O.38:5) | 53.6 |
| LPC(18:1) | 1.3 | PC(38:6) | 22.6 | TAG(50:2) | 48.5 |
| PC(32:1) | 1.3 | TAG(52:2) | 15.4 | PC(32:1) | 47.0 |
| PC(O.34:1) | 1.3 | PC(32:0) | 11.5 | TAG(54:3) | 46.1 |

Age, sex and statin medication were included in all models. ***LASSO:*** All 135 lipids were standardized to have unit variance. One thousand repetitions of L1-regularized Cox regression with the optimal degree of regularization determined by partial likelihood cross-validation were performed. Inclusion fractions refer to the fraction of models in which the respective lipid had a nonzero coefficient. ***BEST) SUBSET:*** Pre-screening was performed on all 135 lipids by backwards stepwise Cox regression and reduced the number of candidate lipids to 46. Cox models were then fitted to all combinations of up to six of these 46. Of the resulting 10.9 million models, the 1000 with the lowest BIC were selected. Inclusion fractions refer to the fraction of models containing the respective lipid. ***STEPWISE:*** For each of 1000 bootstrap resamples (a) all 135 lipids were pre-screened using Cox regression, eliminating lipids with a P < 0.05 and (b) backwards stepwise Cox regression with minimum AIC as selection criterion was performed on the remaining lipids. Inclusion fractions refer to the fraction of the 1000 final models containing the respective lipid. For each selection approach, the ten most frequently selected lipids are shown. The **key selection of LASSO** was well represented within these choices of the other two approaches.

## Claims

1. A method of determining cardiovascular disease (CVD) risk in a subject, comprising:
(a) determining levels of lipids in a sample obtained from the subject, wherein the lipids comprise at least 2 of the following:
(i) a triacylglycerol (TAG) selected from the group consisting of TAG(50:1), TAG(50:2), TAG(50:3), TAG(52:2), TAG(52:3), TAG(52:4), TAG(52:5), TAG(54:2) and TAG(54:3);
(ii) a cholesterol ester (CE) selected from the group consisting of CE(14:0), CE(16:0) and CE(16:1); and
(iii) a phosphatidylethanolamine (PE) selected from the group consisting of PE(34:1), PE(34:2), PE(36:2), PE(36:3), PE(36:4), PE(36:5), PE(38:3), PE(38:4), PE(38:5) and PE(38:6); and
(b) comparing the levels of the lipids in the sample from the subject to control values;
wherein increased levels of the lipids in the sample from the subject compared to the control values indicate an increased risk of CVD in the subject; and
wherein the CVD is associated with atherosclerotic plaque rupture or thrombosis, and/or the CVD is an acute ischemic event selected from myocardial infarction, ischemic stroke or sudden cardiac death.

2. A method according to claim 1, wherein increased levels of the lipids in the sample from the subject compared to the control values are indicative of long-term CVD risk in the subject, the long-term CVD risk comprising the risk of an acute ischemic event occurring in the subject within a period of up to 10 years from when the sample was obtained.

3. A method according to claim 1 or claim 2, wherein the triacylglycerol is TAG(54:2).

4. A method according to any of claims 1 to 3, wherein the cholesterol ester is CE(16:1).

5. A method according to any of claims 1 to 4, wherein the phosphatidylethanolamine is is PE(36:5).

6. A method according to any of claims 1 to 5, wherein the method comprises determining a level of TAG(54:2), CE(16:1) and PE(36:5).

7. A method according to any of claims 1 to 6, further comprising determining a level of a phosphatidylcholine selected from the group consisting of PC(32:0), PC(32:1), PC(34:1), PC(34:3), PC(36:1), PC(36:3), PC(38:2), PC(38:3), PC(38:4), PC(40:2), PC(40:3) and PC(40:4) in the sample from the subject, and comparing the level to a control value, wherein an increased level of the phosphatidylcholine in the sample from the subject compared to the control value indicates an increased risk of CVD in the subject.

8. A method according to any of claims 1 to 7, wherein the sample comprises serum or plasma obtained from the subject.

9. A method according to any of claims 1 to 8, wherein the control values are based on mean levels of the lipids in a control population of subjects.

10. A method according to any of claims 1 to 9, wherein the lipid levels are determined by mass spectrometry.

11. An anti-CVD therapeutic for use in a method of preventing or treating cardiovascular disease in a subject, wherein the method comprises the step of identifying the subject as having an increased risk of developing CVD by the method as defined in any one of claims 1 to 10, and wherein the anti-CVD therapeutic agent is an HMG CoA reductase inhibitor.

## Patentansprüche

1. Verfahren zum Bestimmen des Risikos einer Herz-Kreislauf-Erkrankung (CVD) bei einem Patienten, umfassend:
(a) Bestimmen des Lipidgehalts in einer von dem Patienten erhaltenen Probe, wobei die Lipide mindestens 2 der Folgenden umfassen:
(i) ein Triacylglycerin (TAG), ausgewählt aus der Gruppe bestehend aus TAG(50:1), TAG(50:2), TAG(50:3), TAG(52:2), TAG(52:3), TAG(52:4), TAG(52:5), TAG(54:2) und TAG(54:3);
(ii) einen Cholesterinester (CE), ausgewählt aus der Gruppe bestehend aus CE(14:0), CE(16:0) und CE(16:1); und
(iii) ein Phosphatidylethanolamin (PE), ausgewählt aus der Gruppe bestehend aus PE(34:1), PE(34:2), PE(36:2), PE(36:3), PE(36:4), PE(36:5), PE(38:3), PE(38:4), PE(38:4), PE(38:5) und PE(38:6); und
b) Vergleichen der Lipidgehalte in der Probe des Patienten mit Kontrollwerten;
wobei erhöhte Konzentrationen der Lipide in der Probe des Patienten im Vergleich zu den Kontrollwerten ein erhöhtes Risiko einer CVD bei dem Patienten anzeigen; und
wobei die CVD mit atherosklerotischem Plaque-Bruch oder Thrombose assoziiert ist, und/oder die CVD ein akutes ischämisches Ereignis, ausgewählt aus Myokardinfarkt, ischämischem Schlaganfall oder plötzlichem Herztod, ist.

2. Verfahren nach Anspruch 1, wobei erhöhte Lipidgehalte in der Probe des Patienten im Vergleich zu den Kontrollwerten ein langfristiges CVD-Risiko des Patienten anzeigen, das langfristige CVD-Risiko umfasst das Risiko eines akuten ischämischen Ereignisses, das beim Patienten innerhalb eines Zeitraums von bis zu 10 Jahren ab dem Zeitpunkt, an dem die Probe entnommen wurde, auftritt.

3. Verfahren nach Anspruch 1 oder Anspruch 2, wobei das Triacylglycerin TAG(54:2) ist.

4. Verfahren nach einem der Ansprüche 1 bis 3, wobei der Cholesterinester CE(16:1) ist.

5. Verfahren nach einem der Ansprüche 1 bis 4, wobei das Phosphatidylethanolamin PE(36:5) ist.

6. Verfahren nach einem der Ansprüche 1 bis 5, wobei das Verfahren Bestimmen eines Gehalts an TAG(54:2), CE(16:1) und PE(36:5) umfasst.

7. Verfahren nach einem der Ansprüche 1 bis 6, ferner umfassend Bestimmen eines Gehalts eines Phosphatidylcholins, ausgewählt aus der Gruppe bestehend aus PC(32:0), PC(32:1), PC(34:1), PC(34:3), PC(36:1), PC(36:3), PC(38:2), PC(38:3), PC(38:4), PC(40:2), PC(40:3) und PC(40:4) in der Probe des Patienten, und Vergleichen des Gehalts mit einem Kontrollwert, wobei ein erhöhter Gehalt des Phosphatidylcholins in der Probe des Patienten im Vergleich zu dem Kontrollwert ein erhöhtes CVD-Risiko des Patienten anzeigt.

8. Verfahren nach einem der Ansprüche 1 bis 7, wobei die Probe Serum oder Plasma, das von dem Patienten erhalten wurde, umfasst.

9. Verfahren nach einem der Ansprüche 1 bis 8, wobei die Kontrollwerte auf mittleren Werten der Lipide in einer Kontrollgruppe von Patienten beruhen.

10. Verfahren nach einem der Ansprüche 1 bis 9, wobei die Lipidwerte mittels Massenspektrometrie bestimmt werden.

11. Anti-CVD-Therapeutikum zur Verwendung in einem Verfahren zur Prävention oder Behandlung einer Herz-Kreislauf-Erkrankung in einem Patienten, wobei das Verfahren den Schritt umfasst Identifizieren des Patienten mit einem erhöhten Risiko der Entwicklung von CVD nach dem Verfahren, wie es in einem der Ansprüche 1 bis 10, definiert ist, und wobei das Anti-CVD-therapeutische Mittel ein HMG CoA-Reduktase-Inhibitor ist.

## Revendications

1. Procédé de détermination d'un risque de maladie cardiovasculaire (MCV) chez un sujet, comprenant :
(a) la détermination de niveaux de lipides dans un échantillon obtenu auprès du sujet, où les lipides comprennent au moins 2 des suivants :
(i) un triacylglycérol (TAG) choisi dans le groupe consistant en TAG(50:1), TAG(50:2), TAG(50:3), TAG(52:2), TAG(52:3), TAG(52:4), TAG(52:5), TAG(54:2) et TAG(54:3) ;
(ii) un ester de cholestérol (CE) choisi dans le groupe consistant en CE(14:0), CE(16:0) et CE(16:1) ; et
(iii) une phosphatidyléthanolamine (PE) choisie dans le groupe consistant en PE(34:1), PE(34:2), PE(36:2), PE(36:3), PE(36:4), PE(36:5), PE(38:3), PE(38:4), PE(38:5) et PE(38:6) ; et
(b) la comparaison des niveaux des lipides dans l'échantillon provenant du sujet à des valeurs témoins ;
où des niveaux accrus des lipides dans l'échantillon provenant du sujet comparés aux valeurs témoins indiquent un risque accru de MCV chez le sujet ; et
où la MCV est associée avec une rupture de plaque athérosclérotique ou une thrombose, et/ou la MCV est un événement ischémique aigu choisi parmi l'infarctus du myocarde, l'attaque ischémique ou la mort cardiaque soudaine.

2. Procédé selon la revendication 1, où des niveaux accrus des lipides dans l'échantillon provenant du sujet comparés aux valeurs témoins sont une indication d'un risque de MCV à long terme chez le sujet, le risque de MCV à long terme comprenant le risque d'un événement ischémique aigu survenant chez le sujet dans une période de jusqu'à 10 ans à partir du moment où l'échantillon a été obtenu.

3. Procédé selon la revendication 1 ou la revendication 2, où le triacylglycérol est TAG(54:2).

4. Procédé selon l'une quelconque des revendications 1 à 3, où l'ester de cholestérol est CE(16:1).

5. Procédé selon l'une quelconque des revendications 1 à 4, où la phosphatidyléthanolamine est PE(36:5).

6. Procédé selon l'une quelconque des revendications 1 à 5, où le procédé comprend la détermination d'un niveau de TAG(54:2), CE(16:1) et PE(36:5).

7. Procédé selon l'une quelconque des revendications 1 à 6, comprenant en outre la détermination d'un niveau d'une phosphatidylcholine choisie dans le groupe consistant en PC(32:0), PC(32:1), PC(34:1), PC(34:3), PC(36:1), PC(36:3), PC(38:2), PC(38:3), PC(38:4), PC(40:2), PC(40:3) et PC(40:4) dans l'échantillon provenant du sujet, et la comparaison du niveau à une valeur témoin, où un niveau accru de la phosphatidylcholine dans l'échantillon provenant du sujet comparé à la valeur témoin indique un risque accru de MCV chez le sujet.

8. Procédé selon l'une quelconque des revendications 1 à 7, où l'échantillon comprend du sérum ou du plasma obtenu auprès du sujet.

9. Procédé selon l'une quelconque des revendications 1 à 8, où les valeurs témoins sont basées sur des niveaux moyens des lipides dans une population témoin de sujets.

10. Procédé selon l'une quelconque des revendications 1 à 9, où les niveaux de lipides sont déterminés par spectrométrie de masse.

11. Agent thérapeutique anti-MCV destiné à être utilisé dans un procédé de prévention ou de traitement d'une maladie cardiovasculaire chez un sujet, où le procédé comprend l'étape d'identification du sujet comme ayant un risque accru de développer une MCV par le procédé tel que défini dans l'une quelconque des revendications 1 à 10, et où l'agent thérapeutique anti-MCV est un inhibiteur de HMG CoA réductase.
